# FASCICULE DE BREVET EUROPEEN

(11) **EP 1 644 344 B1**
(45) Date de publication et mention de la délivrance du brevet: **14.09.2011**
(21) Numéro de dépôt: 04785719.8
(22) Date de dépôt: 04.06.2004
(51) Int. Cl.: C07D 257/02, C07D 487/22, C07D 487/18, C07D 487/08

(54) **PROCEDE DE PREPARATION DU CIS-8B-METHYLDECAHYDRO-2A,4A,6A,8A-TETRAAZACYCLOPENTA[FG] ACENAPHTHYLENE, DU CIS-DECAHYDRO-2A,4 A,6A,8A-TETRAAZACYCLOPENTA[FG] ACENAPHTHYLENE, DU CYCLENE, ET DE CYCLENES FONCTIONNALISES**
VERFAHREN ZUR HERSTELLUNG VON CIS-8B-METHYLDECAHYDRO-2A,4A,6A,8A-TETRAAZACYCLOPENTA[FG]ACENAPHTHYLEN, CIS-DECAHYDRO-2A,4A,6A,8A-TETRAAZACYCLOPENTA[FG]ACENAPHTHYLEN, CYCLEN UND FUNKTIONALISIERTEN CYCLENEN
METHOD OF PREPARING CIS-8B-METHYLDECAHYDRO-2A,4A,6A,8A-TETRAAZACYCLOPENTA [FG]ACENAPHTHYLENE, CIS-DECAHYDRO-2A,4A,6A,8A-TETRAAZACYCLOPENTA [FG]ACENAPHTHYLENE, CYCLENE AND FUNCTIONALISED CYCLENES

(30) Priorité: 13.06.2003 FR 0350217
(43) Date de publication de la demande: 12.04.2006
(73) Titulaire: CENTRE NATIONAL DE LA RECHERCHE SCIENTIFIQUE (CNRS), 75016 Paris (FR)
(72) Inventeur: BOSCHETTI, Frédéric, F-21000 DIJON (FR); CHAUX, Fanny, F-21000 DIJON (FR); DENAT, Franck, F-21000 DIJON (FR); GUILARD, Roger, F-21121 FONTAINE-LES-DIJON (FR); LEDON, Henry, F-78000 VERSAILLES (FR)
(74) Mandataire: Colombet, Alain André
(86) Numéro de dépôt international: PCT/FR2004/050213
(87) Numéro de publication internationale: WO 2005/000823

(56) Documents cités:
- WO-A-00/32601
- WO-A-02/06287
- WO-A-97/49691
- WO-A-03/029228
- HUBIN, TIMOTHY J. ET AL: "Synthesis and X-ray Crystal Structure Determination of the First Copper(II) Complexes of Tetraazamacrocycle-Glyoxal Condensates" INORGANIC CHEMISTRY, 41(26), 7006-7014, 2002, XP002269063
- HERVE, GWENAELLE ET AL: "A new route to cyclen, cyclam and homocyclen" TETRAHEDRON LETTERS, 39(38), 6861-6864, 1998, XP004132624 cité dans la demande
- SANDNES, ROLF WIGGO ET AL: "A simple synthesis of the macrocycle 1,4,7,10-tetraazacyclododecane" ACTA CHEMICA SCANDINAVICA, 52(12), 1402-1404, 1998, XP009025163 cité dans la demande

## Description

L'invention à pour objet une nouvelle synthèse de cycles poly-azotés ainsi que certains de ces cycles.

Parmi les polyamines cycliques, le cyclène ou 1,4,7,10-tétraazacyclododécane, est l'un des composés les plus étudiés car il est le produit de départ de la synthèse de nombreux complexes utilisés dans les sondes luminescentes, comme vecteurs de radioéléments en radio-immunothérapie ou encore comme agents de contraste dans l'imagerie médicale. Dans cette dernière application, on utilise très largement des complexes de gadolinium (III) dont le ligand est un cyclène poly fonctionnalisé par des bras carboxylates. Les produits les plus connus en Europe sont le Dotarem™ commercialisé par la société Guerbet, le ProHance™ commercialisé par la société Bracco, et le Gadovist™ commercialisé par la société Schering.

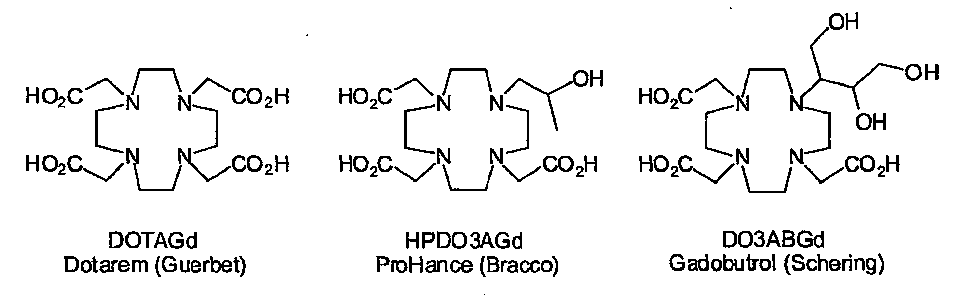

L'intérêt économique de ces systèmes a logiquement suscité de nombreux travaux de recherche en amont, pour développer de nouvelles voies de synthèse de ces ligands, qui ont donné lieu à de nombreuses publications scientifiques et demandes de brevets, référencées 1 à 39 à la fin de cet exposé.

Si le Dotarem™, qui est un complexe du gadolinium (III) ou Gd(III) et de l'acide 1,4,7,10-tétraazacyclododécane-1,4,7,10-tétracétique, ou DOTA, est préparé en une seule étape à partir du cyclène, en revanche, les autres complexes ayant des groupes fonctionnels différents sont préparés par des méthodes de synthèse plus délicates qui nécessitent la mise au point de procédés de fonctionnalisation sélective du macrocycle.

Or les méthodes utilisées jusqu'à présent, ne sont pas satisfaisantes en ce que, soit elles impliquent l'utilisation en large excès de macrocycles afin de minimiser les produits polysubstitués indésirables, soit elles incluent des séquences successives de protection puis de dé-protection des sites réactifs.

Les méthodes de préparation du cyclène les plus performantes sont des variantes effectuées selon le schéma suivant :

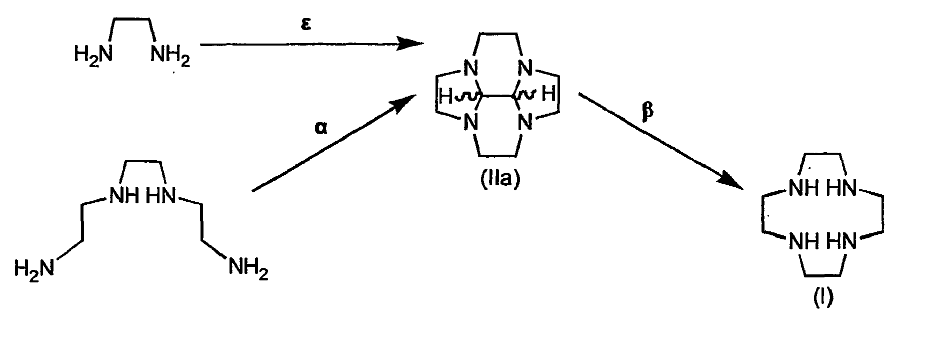

### Etape α :

Selon la référence 1:1. (MeO)₂CHNMe₂ (30 mn au reflux) ; 2. BrCH₂CH₂Br, Na₂CO₃, MeCN (3h au reflux) ; 3. H₂O₂, eau ; 4. NaBH₄ (excès) dans EtOH ;
   Rendement global : 52% ;
Selon les références 2 et 9 : 1. glyoxal, EtOH (12 h à température ambiante) ;
   2. BrCH₂CH₂Br, DMF (20 h à Température ambiante) ; Rendements globaux : 70%
   (référence 2) et 28% (référence 9) ;
Selon les références 4, 6 et 12 : 1. glyoxal, Ca(OH)₂, eau (2 h à 5°C) ; 2. ClCH₂CH₂Cl ou BrCH₂CH₂Br ou ClCH₂CH₂Cl + NaBr, Na₂CO₃, DMAC (48 h à 50°C) ; (+H₃PO₄/eau dans référencel2) ; Rendement globaux : 33% (référence 4), 62% (référence 6) et 58% (référence 12) ;
Selon la référence 17:1. glyoxal, Ca(OH)₂, eau ; 2. (CO₂Et)₂, EtOH, présence ou non de 2-hydroxypyridine ou méthanoate de sodium ou bien chloroacétate d'éthyle en présence de Na₂CO₃ et NaI dans EtOH ; 3. reducteur : Red-A1 ou vitride, toluène (112°C) ; divers modes opératoires pour les étapes 2 et 3 ; Rendement global : 50% maximum ;

### Etape β :

Selon la référence 2 : 10 eq. NH₂OH, EtOH (16 h au reflux) ; Rendement : 80% ;
Selon la référence 4 : Br₂ ou KMnO₄ ou NaOCl puis H₂O/NaOH (150°C à 180°C en autoclave) ; Rendement : 38 à 68% ; ou bien H₂SO₄ 50% (24 h à 112°C) ; Rendement : 22%;
Selon la référence 13 : 3 eq. Br₂ puis H₂O/NaOH (36 h au reflux) ; Rendement = 65% ;
Selon la référence 12 : diéthylènetriamine, eau/HCl (24 h au reflux) puis traitements successifs par RCl ; Rendement : 36% ;

### Etape ε :

Selon la référence 13 : 1. 0,17 eq. glyoxal, EtOH (30 mn au reflux) ; 2. 6 eq. ClCH₂CH₂Cl, Na₂CO₃, DMAC (24 h à 70°C) ; Rendement global : 60%.

Selon un premier aspect, l'invention a pour objet un procédé de préparation du cyclène de formule (I) : 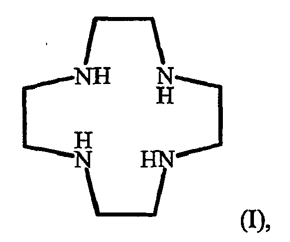 à partir de triéthylènetétraamine de formule (VIII) : 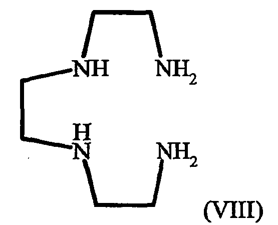 ou d'éthylènediamine de formule (VIII') : 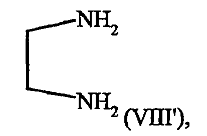 caractérisé en ce qu'il est constitué de l'une ou l'autre des séries d'étapes successives suivantes :

La série d'étapes (I), constituée d'une étape (A) de préparation en un seul pot (dite "one pot" en langue anglaise) du composés de formule (IIa) : 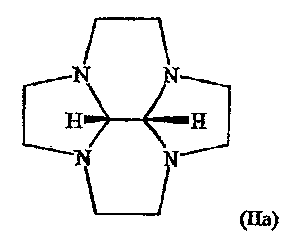 à partir du composé de formule (VIII) telle que définie précédemment, suivie d'une étape (B) de transformation du composé de formule (IIa), en cyclène de formule (I) ;

La série d'étapes (II), constituée d'une étape (C) de préparation du composé de formule (IIb) : 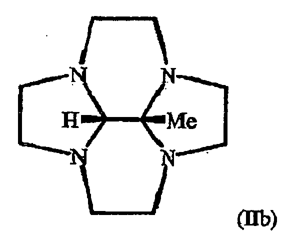 à partir du composé de formule (VIII) telle que définie précédemment, suivie d'une étape (D) de transformation du composé de formule (IIb), en cyclène de formule (I) ; ou

La série d'étapes (III), constituée d'une étape (E) de préparation en un seul pot (dite "one pot" en langue anglaise) du composé de formule (IIa) telle que définie précédemment, à partir du composé de formule (VIII) telle que définie précédemment, suivie d'une étape (B) de transformation du composé de formule (IIa), en cyclène de formule (I).

Les étapes (A), (C) et (E) sont caractérisées en ce qu'elles sont effectuées en l'absence de dérives halogénés.

Les conditions expérimentales adaptées à la préparation du cyclènes à partir de la triéthylènetétraamine de formule (VIII) ou de l'éthylènediamine de formule (VIII'), par un des deux intermédiaires "bisaminal" de formule (IIa) ou de formule (IIb), sont répertoriées sous le schéma suivant:

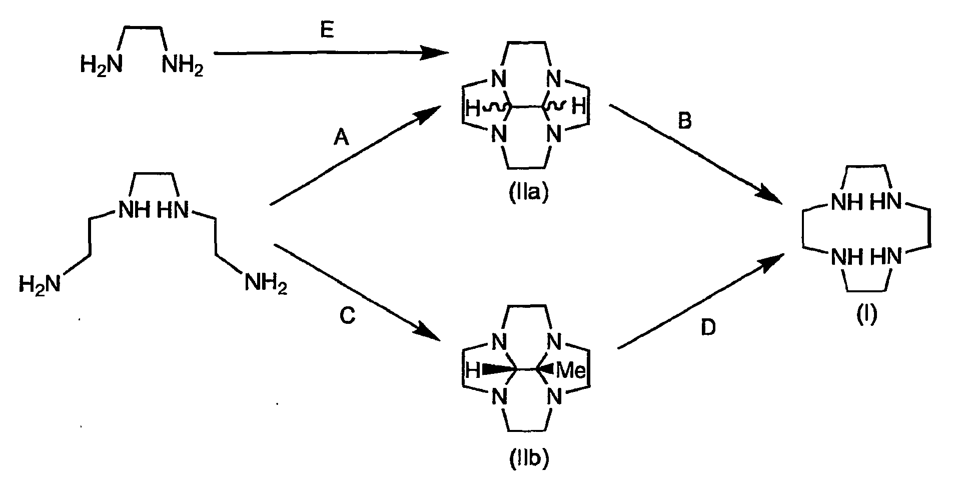

### Etape A :

2 équivalents de glyoxal, 2 équivalents de benzotriazole, eau/MeOH (4 h à température ambiante), puis NaBH₄ (2 h à température ambiante) ; Rendement : 68 %.

### Etape B :

10 équivalents NH₂OH, EtOH (16 h au reflux) ou
Br₂ ou KMnO₄ ou NaOCl puis H₂O / NaOH (150°C à 180°C en autoclave) ou
Br₂ ou KMnO₄ ou NaOCl puis H₂SO₄ 50% (24 h à 112°C) ou
3 équivalents Br₂ puis H₂O / NaOH (36 h au reflux) ou

Diéthylènetriamine, eau / HCl (24 h au reflux) puis traitements successifs par HCl.

### Etape C :

Pyruvaldéhyde, eau (2 h à 2°C) puis glyoxal, 2 équivalents de benzotriazole, eau / MeOH (2 h à température ambiante), puis NaBH₄ (2 h à température ambiante) ou
Pyruvaldéhyde, eau (2 h à 2°C) puis glyoxal, 2 équivalents de benzotriazole, eau / EtOH (2 h à température ambiante), puis NaBH₄ (0°C puis 2 h à température ambiante) ou
Pyruvaldéhyde, EtOH (2 h à 0°C) puis glyoxal, 2 équivalents de benzotriazole, eau / EtOH (2 h à température ambiante), puis NaBH₄ (0°C puis 2 h à température ambiante) ou
Pyruvaldéhyde, eau (2 h à 2°C) puis BrCH₂CH₂Br, K₂CO₃, MeCN (48 h à 60°C).

### Etape D :

HCl 37% (12h au reflux).

### Etape E :

0,5 équivalent de glyoxal, eau (1 h à 2°C) puis 1 équivalent de glyoxal, 4 équivalents de benzotriazole, eau/MeOH (2 h à température ambiante) puis NaBH₄ (2 h à température ambiante).

On peut également mentionner un procédé dans lequel la série d'étapes (II) est constituée d'une étape (C₁) de préparation du composé de formule (III): 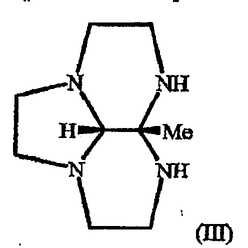 à partir du composé de formule (VIII) telle que définie précédemment, suivie d'une étape (C₂) de transformation du composé de formule (III) en composé de formule (IIb) telle que définie précédemment, suivie d'une étape (D) de transformation du composé de formule (IIb), en cyclène de formule (I).

Selon une variante du procédé tel que défini ci-dessus, la série d'étape (II) est réalisée enun seul pot

Selon un deuxième aspect de la présente invention celle-ci a pour objet le composé de formule (IIb) telle que définie précédemment.

On peut également mentionner un procédé de préparation de dérivés du cyclène N-mono fonctionnalisés de formule (Va) : 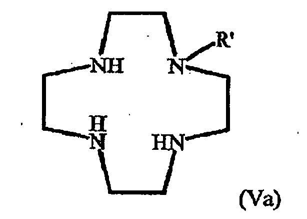 et de dérivés du cyclène N₁,N₇-difonctionnalisés de formule (Vb) : 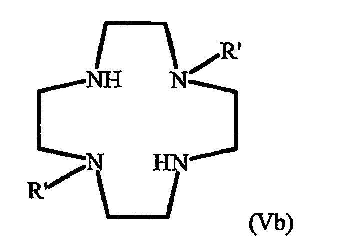 formules (Va) et (Vb) dans lesquelles R' représente :
- un radical alkyle, linéaire ou ramifié, comportant de 1 à 15 atomes de carbone ou,
- un radical -(CH₂)_{w}-Y, dans lequel w représente un nombre supérieur à zéro et inférieur ou égal à 6 et plus particulièrement inférieur ou égal à 3, et
- soit Y représente un cycle aromatique comportant de 6 à 14 atomes de carbone, éventuellement substitué en position(s) ortho et/ou meta et/ou para ou bien par un atome d'halogène ou bien par un groupe alkyle comportant de 1 à 4 atomes de carbone ou bien par un radical OH ou bien par un radical OR¹ dans lequel R' représente un radical alkyle comportant de 1 à 4 atomes de carbone, un radical aryle ou un hétérocycle aromatique ou bien par un groupe nitro ou bien par un groupe NH₂, -(C=O)NH₂, NHR², -(C=O)NHR², NR²R³ ou -(C=O)NR²R³, dans lesquels R² et R³ identiques ou différents, représentent indépendamment l'une de l'autre, un radical alkyle choisi parmi les radicaux méthyle, éthyle, propyle, isopropyle, n-butyle, isobutyle, sec-butyle ou tert-butyle,
- soit Y représente un hétérocycle aromatique et plus particulièrement un hétérocycle comportant de un à 3 atomes d'azote, de 4 à 12 atomes de carbone, éventuellement substitué en position(s) ortho et/ou méta et/ou para ou bien par un atome d'halogène ou bien par un groupe alkyle comportant de 1 à 4 atomes de carbone ou bien par un radical OH ou bien par un radical OR¹ dans lequel R¹ représente un radical alkyle comportant de 1 à 4 atomes de carbone, un radical aryle ou un hétérocycle aromatique ou bien par un groupe nitro ou bien par un groupe NH₂, -(C=O)NH₂, NHR², - (C=O)NHR², NR²R³ ou -(C=O)NR²R³" dans lesquels R² et R³ identiques ou différents, représentent indépendamment l'une de l'autre, un radical alkyle choisi parmi les radicaux méthyle, éthyle, propyle, isopropyle, n-butyle, isobutyle, sec-butyle ou tert-butyle,
- soit Y représente un radical choisi parmi les radicaux cyano, PO₃H₂, SO₃H, NH₂, -(C=O)NH₂, NHR², -(C=O)NHR², NR²R³ ou -(C=O)NR²R³, dans lesquels R² et R³ identiques ou différents, représentent indépendamment l'un de l'autre, un radical alkyle choisi parmi les radicaux méthyle, éthyle, propyle, isopropyle, n-butyle, isobutyle, sec-butyle ou tert-butyle ou un radical CO₂R⁴, dans lequel R⁴ représente un atome d'hydrogène, un radical alkyle comportant de 1 à 4 atomes de carbone ou un radical aryle et plus particulièrement un radical phényle,
caractérisé en ce que le composé de formule (IX) :

R'X (IX)

dans laquelle X représente un atome d'halogène et R' est tel que défini précédemment est mis à réagir avec le composé de formule (IIb) telle que définie précédemment pour conduire selon la stoechiométrie de la réaction, soit au mélange des composés de formules (IVa) : 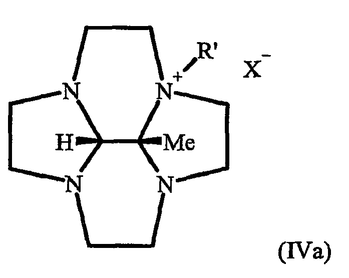 et (IV'a) : 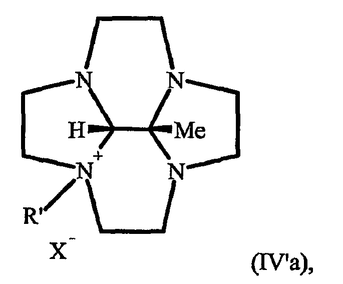 soit au composé de formule (IVb) : 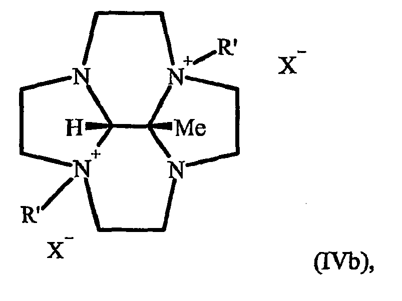 et on ce que lesdits composés de formules (IVa), (IVa) ou ledit composé de formule (IVb), sont soumis à une hydrolyse alcaline pour conduire respectivement au composé de formules (Va) ou (Vb).

On peut également mentionner un procédé de préparation de composés de formule (VI) : 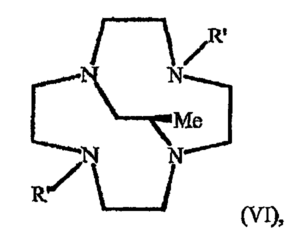 dans laquelle R' est tel que défini précédemment, par réduction du composé de formule (IVb).

On peut également mentionner un procédé de réparation de composés de formule (VII) : 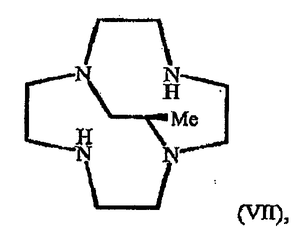 par hydrogénolyse du composé de formule (VI) toile que définie précédemment.

Selon un dernier aspect, l'invention a pour objet les composés de formules (IVa), (IV'a),(IVb),(VI) et (VII).

Les exemples suivants illustrent l'invention sans toutefois la limiter.

### Exemple 1 Synthèse du décahydro-2a,4a,6a,8a-tétraaza-cyclopenta[fg]acénaphthylène [composé de formule (IIa) ; série d'étapes (I), étape (A)]

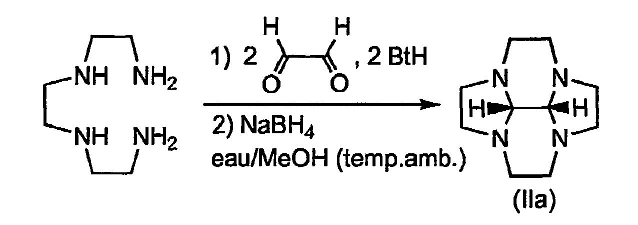

A une solution de 10,00 g (68,4 mmol) de triéthylènetétraamine et 16,30 g (136,7 mmol) de benzotriazole (BtH) dans 200 ml d'eau distillée refroidie à 2°C est ajoutée par un lent goutte-à-goutte une solution de 19,86 g (136,7 mmol) de glyoxal (40 % dans l'eau) dans 80 ml de méthanol. A la fin de l'addition, le mélange est porté à température ambiante et il est agité pendant 4h. 5,18 g (136,7 mmol) de borohydrure de sodium (NaBH₄), sont alors ajoutés par petites quantités. Après 2h d'agitation à température ambiante, le méthanol est évaporé et 13 g de pastilles de potasse (KOH) sont ajoutés. La solution est alors extraite par 3 fois 400 ml de chloroforme. Après séchage sur sulfate de magnésium (MgSO₄) et évaporation des solvants, le composé (IIa) est isolé sous la forme d'une huile jaune qui cristallise (9,00 g ; Rdt = 68 %).
RMN ¹³C (125 MHz, CDCl₃, δ en ppm) : 50,9 ; 51,7 ; 78,1.

### Exemple 2: Synthèse du décahydro-2a,4a,6a,8a-tétraazacyclopenta[fg]acénaph thylène [composé de formule (IIa) ; série d'étapes (III), étape (E)]

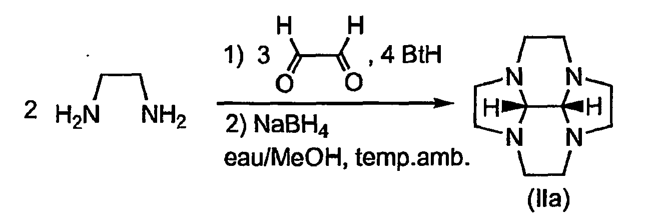

A une solution de 0,82 g (13,6 mmol) d'éthylènediamine, dans 20 ml d'eau distillée refroidie à 2°C est ajoutée par un lent goutte-à-goutte une solution de 0,99 g (6,8 mmol) de glyoxal (40 % dans l'eau) dans 5 ml d'eau. A la fin de l'addition, le mélange est porté à température ambiante et est agité pendant 1h. 3,25 g (27,3 mmol) de benzotriazole sont alors ajoutés et une solution de 1,98 g (13,6 mmol) de glyoxal (40 % dans l'eau) dans 20 ml de méthanol est additionnée. Après 2h d'agitation à température ambiante, 1,03 g (27,3 mmol) de NaBH₄ sont additionnés par petites quantités. Après 2h d'agitation, le méthanol est évaporé et 2 g de pastilles de KOH sont ajoutés. La solution est alors extraite par 3 fois 100 ml de chloroforme. Après séchage sur MgSO₄ et évaporation des solvants, (IIa) est isolé sous la forme d'une huile jaune qui cristallise (0,4 g ; Rdt = 30%).

### Exemple 3 : Synthèse du 8b-méthyl décahydro-2a,4a,6a,8a-tetraazacyclopenta[fg] acénaphthylène [composé de formule (IIb) ; série d'étapes (II) ; étapes (C₁) et (C₂)]

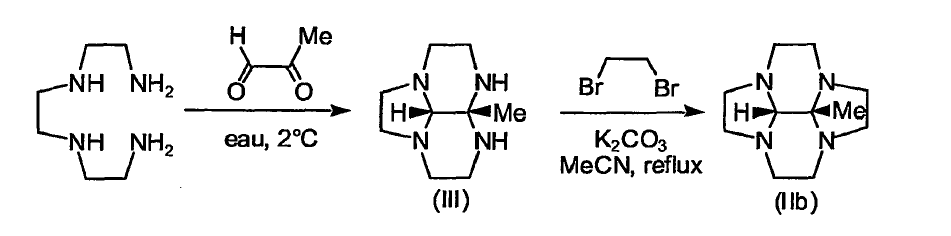

### Synthèse du composé de formule (III) [étape (C₁)]

A une solution de 1,00 g (6,8 mmol) de triéthylènetétraamine dans 40 ml d'eau distillée refroidie à 2°C est ajoutée par un lent goutte-à-goutte une solution de 1,23 g (6,8 mmol) de pyruvaldéhyde (40 % dans l'eau) dans 10 ml d'eau. A la fin de l'addition, le mélange réactionnel est maintenu à 2°C pendant 2h. L'eau est ensuite évaporée sous pression réduite et le composé (III) est obtenu sous la forme d'une huile orange utilisée sans purification pour la synthèse du composé (IIb) (1,25 g).

### Synthèse du composé de formule (IIb) [étape (C₂)]

Une solution de 1,25 g (6,8 mmol) de composé (III) et de 3,79 g (27,4 mmol) de carbonate de potassium (K₂CO₃) dans 20 ml d'acétonitrile, est portée à 60°C. Une solution de 1,38 g (6,8 mmol) de 1,2-dibromoéthane dans 10 ml d'acétonitrile est alors additionnée. Le mélange est agité à 60°C pendant 2 jours. Après filtration et évaporation des solvants, le résidu est repris dans 20 ml d'eau et extrait par 3 fois 100 ml de chloroforme. Après séchage sur MgSO₄ et évaporation des solvants, (IIb) est isolé sous la forme d'une huile orange (0,42 g ; Rdt = 30 %).
RMN ¹³C (125 MHz, CDCl₃, δ en ppm) : 17,9; 47,7; 48,9; 49,3; 51,7 ; 75,8; 82,1.

### Exemple 4 : Synthèse du 8b-méthyl décahydro-2a,4a,6a,8a-tétraazacyclopenta[fg] acénaphthylène [composé de formule (IIb) ; série d'étapes (II) ; étape (C)]

A une solution de 50 g (341,8 mmol) de triéthylènetétraamine dans 800 ml d'eau distillée refroidie à 2°C est ajoutée par un lent goutte-à-goutte une solution de 61,53 g de pyruvaldéhyde à 40 % dans l'eau (341,8 mol) dans 20 ml d'eau. A la fin de l'addition, le mélange réactionnel est porté à température ambiante et 81,36 g (683,6 mmol) de benzotriazole sont ajoutés en une seule fois. Une solution de glyoxal (40 % dans l'eau) (341,8 mmol) dans 200 ml de méthanol est alors additionnée par un lent goutte-à-goutte. Après 2h d'agitation à température ambiante, 25,85 g (683,6 mmol) de NaBH₄ sont additionnés par petites quantités. Après 2h d'agitation à température ambiante, le méthanol est évaporé et 50 g de pastilles de KOH sont ajoutés au résidu. La solution est alors extraite par 3 fois 1,5 1 de chloroforme. Après séchage sur MgSO₄ et évaporation des solvants, (IIb) est isolé sous la forme d'une huile orange (28,50 g ; Rdt = 40 %).

### Exemple 5 : Synthèse du 8b-méthyl décahydro-2a,4a,6a,8a-tétraazacyclopenta[fg] acénaphthylène [composé de formule (IIb) : série d'étapes (II) ; étape (C)]

A une solution de 60,66 g (342 mmol) de triéthylènetétraamine hydrate (contenant 17% d'eau) dans 300 ml d'eau distillée refroidie à 2°C est ajoutée par un lent goutte-à-goutte une solution de 61,56 g de pyruvaldéhyde à 40 % dans l'eau (342 mmol) dans 100 ml d'eau. A la fin de l'addition, le mélange réactionnel est porté à température ambiante et 81,40 g (684 mmol) de benzotriazole sont ajoutés en une seule fois. 300 ml d'éthanol sont alors ajoutés au milieu réactionnel, puis une solution de 49,59 g de glyoxal (40 % dans l'eau) (342 mmol) dans 100 ml d'éthanol est additionnée par un lent goutte-à-goutte. Après 2h d'agitation à température ambiante, la solution est refroidie à 0°C et 25,87 g (684 mmol) de NaBH₄ sont additionnés par petites quantités. Après retour à température ambiante, le milieu réactionnel est agité pendant 2h, l'éthanol est évaporé et 70 g de pastilles de KOH sont ajoutés au résidu. La solution est alors extraite par 2 fois 1 1 de chloroforme. Après séchage sur MgSO₄ et évaporation des solvants, (IIb) est isolé sous la forme d'une huile orange (32,3 g ; Rdt = 45 %).

### Exemple 6 : Synthèse du 8b-méthyl décahydro-2a,4a,6a,8a-tétraazacyclopenta[fg] acénanhthvlène [composé de formule (IIb) : série d'étapes (II) ; étape (C)]

A une solution de 5 g (34,2 mmol) de triéthylènetétraamine dans 30 ml d'éthanol refroidie à 0°C est ajoutée par un lent goutte-à-goutte une solution de 6,16 g de pyruvaldéhyde à 40 % dans l'eau (34,2 mmol) dans 10 ml d'éthanol. A la fin de l'addition, le mélange réactionnel est porté à température ambiante et 8,15 g (68,5 mmol) de benzotriazole sont ajoutés en une seule fois. Une solution de 4,97 g de glyoxal (40 % dans l'eau) (34,2 mmol) dans 5 ml d'éthanol est additionnée par un lent goutte-à-goutte. Après 2h d'agitation à température ambiante, la solution est refroidie à 0°C et 2,59 g (68,5 mmol) de NaBH₄ sont additionnés par petites quantités. Après retour à température ambiante, le milieu réactionnel est agité pendant 2 heures, l'éthanol est évaporé, 50 ml d'eau et 6,5 g de pastilles de KOH sont ajoutés au résidu. La solution est alors extraite par 2 fois 300 ml de chloroforme. Après séchage sur MgSO₄ et évaporation des solvants, (IIb) (un mélange de plusieurs isomères) est isolé sous la forme d'une huile orange (4,84 g ; Rdt = 68 %).

### Exemple 7: Synthèse du cyclène [composé de formule (I); Série d'étapes (II); étape (D)]

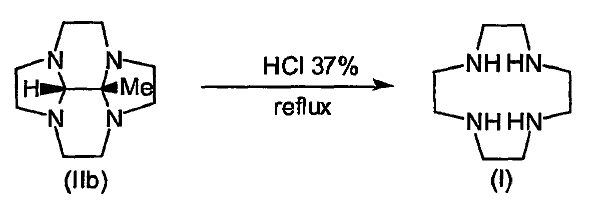

1,00g de composé (IIb) préparé selon l'exemple 4 est placé dans 20 ml d'HCl à 37 %. Le mélange est porté à reflux pendant 12 h. Après refroidissement et filtration, Le tétrachlorhydrate du composé (I), est isolé sous la forme d'une poudre blanche (0,75 g ; Rdt = 50 %).
RMN¹³C (125 MHz, D₂O, δ en ppm) : 44,6.

### Exemple 8 : Synthèse du cyclène [composé de formule (I) : Série d'étanes(II) : étapes (C) + (D) "en un seul pot"]

A une solution de 581 g (3,1 mol) de triéthylènetétraamine hydraté (contenant 22% d'eau) dans 3,5 1 d'eau distillée refroidie à 2°C est ajoutée par un lent goutte-à-goutte une solution de 555 g de pyruvaldéhyde à 40 % dans l'eau (3,1 mal) dans 500 ml d'eau. A la fin de l'addition, le mélange réactionnel est porté à température ambiante et agité pendant deux heures. 739 g (6,2 mol) de benzotriazole dans 3,5 1 d'éthanol sont ajoutés en une seule fois, puis une solution de 450 g de glyoxal (40 % dans l'eau) (3,1 mol) est additionnée par un lent goutte-à-goutte. Après 12 h d'agitation à température ambiante, la solution est refroidie à 0°C et 235 g (6,2 mol) de NaBH₄ sont additionnés par petites quantités. Après retour à température ambiante, le milieu réactionnel est agité pendant 2h. On filtre le précipité formé et on réduit le volume du milieu réactionnel à 3 1. Après extraction au chloroforme, séchage sur sulfate de magnésium et évaporation des solvant, (IIb) est isolé sous la forme d'une huile orange qui est placée dans 4 1 d'HCl à 37 %. Le mélange est chauffé à 80°C pendant 12 h. Après refroidissement et filtration, Le tétrachlorhydrate du composé (I), est isolé sous la forme d'une poudre blanche (147g; Rdt= 15 %).

### Exemple 9 : Synthèse d'un mélange d'isomères (IVa) + (IVa') [composés dans lesQuels R' = -CH₂-C₆H₅et X-Br)

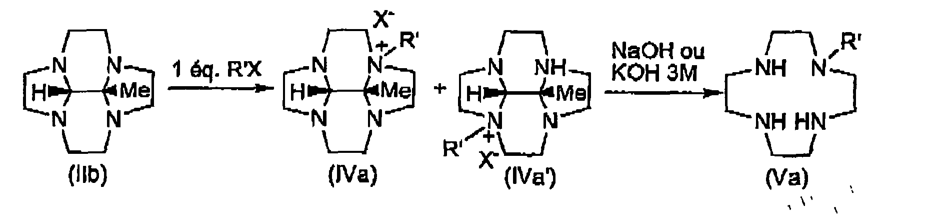

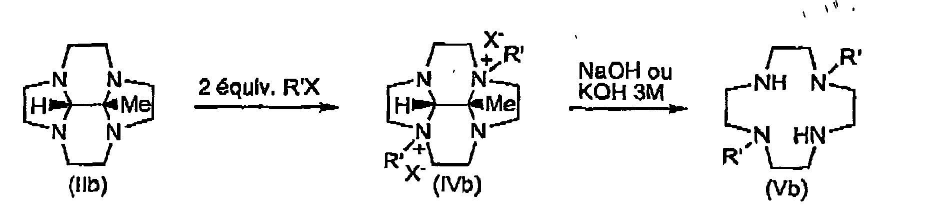

A une solution de 1,01 g (4,86 mmol) de composé (IIb) dans 4 ml de toluène, est ajouté 0,66 g (3,86 mmol) de bromure de benzyle. Après 24 heures d'agitation à température ambiante, le solide formé est filtré, lavé à l'éther puis séché. Le mélange des deux isomères (IVa) et (IVa') (R' = -CH₂-C₆H₅, X = Br) est isolé sous forme d'une poudre jaune (1,24 g ; Rdt= 84 %).
RMN ¹³C (125 MHz, DMSO-d⁶, δ en ppm) : 12,0 ; 25,0 ; 43,0 ; 44,8 ; 45,0 ; 45,4 ; 47,9 ; 48,3 ; 48,6 ; 49,2 ; 49,5 ; 49,6 ; 50,6 ; 52,5 ; 56,7 ; 58,6 ; 59,3 ; 61,1 ; 62,8 ; 76,4 ; 77,7 ; 87,5 ; 87,9 ; 126,3 ; 129,2 ; 129,5 ; 129,9 ; 130,1 ; 131,1 ; 131,3 ; 133,2 ; 134,2.

### Exemple 10 : Synthèse d'un mélange d'isomères (IVa) + (IVa') [composés dans lesquels R' = -CH₂-C₆H₄-pCH=CH₂ et X = I]

A une solution de 1,06 g (5,10 mmol) de composé (IIb) et 0,61 g (4,07 mmol) d'iodure de sodium dans 6 ml d'acétonitrile est ajouté 0,62 g (4,07 mmol) de chlorure de 4-vinylbenzyle. Après 24 heures d'agitation à température ambiante, le solide formé est filtré, lavé à l'éther puis séché. Le mélange des deux isomères (IVa) et (IVa') (R' = - CH₂-C₆H₄-pCH=CH₂, X = I) est isolé sous forme d'une poudre jaune (0,92 g) contenant du chlorure de sodium.
RMN ¹³C (125 MHz, DMSO-d⁶, δ en ppm) : 12,1 ; 25,2 ; 43,0 ; 44,7 ; 45,1 ; 45,5 ; 47,9 ; 48,3 ; 48,6 ; 49,3 ; 49,5 ; 49,7 ; 50,4 ; 52,5 ; 56,8 ; 58,4 ; 59,4 ; 61,1 ; 62,4 ; 76,5 ; 77,7 ; 87,3 ; 88,0 ; 117,2 ; 127,5 ; 127,8 ; 128,9 ; 129,3 ; 133,5 ; 134,5 ; 136,8 ; 139,7 ; 139,9.

### Exemple 11 : Synthèse d'un mélange d'isomères (IVa) + (IVa') [composés dans lesquels R' = -CH₂CONH₂ et X = I)]

A une solution de 0,51 g (2,45 mmol) de composé (IIb) dans 6 ml de tétrahydrofurane est ajouté 0,36 g (1,95 mmol) d'iodoacétamide. Après 24 heures d'agitation à température ambiante, le solide formé est filtré, lavé à l'éther puis séché. Le mélange des deux isomères (IVa) et (IVa') (R'= -CH₂CONH₂, X = I) est isolé sous la forme d'une poudre orange (0,46 g ; Rdt= 60%).
RMN ¹³C (125 MHz, D₂O, δ en ppm) : 11,2 ; 21,5 ; 25,7 ; 41,0 ; 43,9 ; 44,7 ; 45,5 ; 46,3 ; 47,0 ; 47,7 ; 47,9 ; 48,4 ; 49,3 ; 50,6 ; 56,7 ; 58,6 ; 60,2 ; 62,0 ; 65,0 ; 68,3 ; 75,9 ; 77,6 ; 89,1 ;157,0.

### Exemple 12 : Synthèse d'un composé (IVb) [composé dans lequel R' = -CH₂C₆H₅ et X = I)

A une solution de 0,49 g (2,36 mmol) de composé (IIb) et 0,92 g (7,07 mmol) d'iodure de sodium dans 10 ml d'acétonitrile sont ajoutés 1,21 g (7,07 mmol) de bromure de benzyle sans précaution. Après 24 heures d'agitation à température ambiante, le précipité obtenu est filtré, lavé à l'éther puis séché. Le composé (IVb) (R' = -CH₂-C₆H₅, X = I) est isolé sous forme d'une poudre jaune-orangé (1,33 g) contenant du bromure de sodium.
RMN ¹³C (125 MHz, DMSO-d⁶ ; δ en ppm) : 15,7 ; 42,4 ; 45,5 ; 46,2 ; 55,3 ; 55,7 ; 58,5 ; 59,0 ; 61,2 ; 62,4 ; 84,7 ; 86,8 ; 129,4 ; 129,5 ; 130,0 ; 130,4 ; 131,5 ; 131,6 ; 133,3 ; 134,4.

### Exemple 13 : Synthèse d'un composé (Va) dans lequel R' = -CH₂-C₆H₅

Une solution de 1,10 g (2,90 mmol) du mélange d'isomères (IVa) + (IVa') (R' = -CH₂-C₆H₅, X = Br ) préparé selon l'exemple 8 dans 50 ml d'une solution aqueuse d'hydroxyde de potassium 3M est chauffée à 80°C pendant 16 heures. La solution est extraite par trois fois 30 ml de chloroforme. Après séchage sur MgSO₄ et évaporation des solvants, le composé (Va) (R' = -CH₂-C₆H₅) est obtenu sous la forme d'une huile (0,54 g, Rdt= 72 %).
RMN ¹³C (125 MHz, CDCl₃, δ en ppm) : 45,7 ; 46,8 ; 47,8 ; 51,8 ; 59,8 ; 127,5 ; 128,8 ; 129,5, 139,4.

### Exemple 14 : Synthèse du composé (Va) dans lequel R' = -CH₂C₆H₄-CH=CH₂

Une solution de 0,92 g du mélange d'isomères (IVa) + (IVa') (R' = -CH₂-C₆H₄-CH=CH₂, X = I) contenant du chlorure de sodium, préparé selon l'exemple 9, dans 20 ml d'une solution aqueuse d'hydroxyde de potassium 2M est agitée à température ambiante pendant 72h. La solution est extraite par trois fois 20 ml de chloroforme. Après séchage sur MgSO₄ et évaporation des solvants, le composé (Va) (R' = -CH₂-C₆H₄-CH=CH₂) est obtenu sous la forme d'une huile jaune (0,40 g, Rdt global par rapport au chlorure de 4-vinylbenzyle = 34%).
RMN ¹³C (125 MHz, CDCl₃, δ en ppm) : 45,5 ; 46,8 ; 47,6 ; 52,0 ; 59,4 ; 113,8 ; 126,8 ; 129,7 ; 136,9 ; 137,2 ; 139,2.

### Exemple 15 : Synthèse du composé (Vb) dans lequel R' = -CH₂-C₆H₅

Une solution de 1,9 g de composé (IVb) (R' = -CH₂-C₆H₅, X = I) contenant du bromure de sodium, préparé selon l'exemple 11, dans 50 ml d'une solution d'hydroxyde de sodium 3M est portée au reflux pendant 12h. La solution est extraite par trois fois 100 ml de chloroforme. Après séchage sur MgSO₄ et évaporation des solvants, le composé (Vb) (R' = -CH₂-C₆H₅) est obtenu sous la forme d'une huile jaune (0,45 g, Rdt global par rapport au composé (IIb) = 27%).
RMN ¹³C (125 MHz, CDCl₃, δ en ppm) : 45,9 ; 52,6 ; 60,6 ; 127,8 ; 129,0 ; 129,7 ; 139,8.

### Exemple 16 : Synthèse du composé (VI) dans lequel R' = -CH₂-C₆H₅

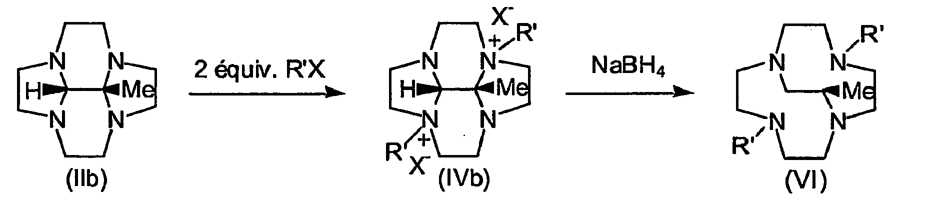

Une solution de 0,75 g (1,16 mmol) du composé (IVb) (R" = -CH₂-C₆H₅, X = I) contenant du bromure de sodium, préparé selon l'exemple 11, dans 25 ml d'éthanol absolu est porté à 0°C et 1,10 g (29,1 mmol) de borohydrure de sodium sont ajoutés lentement. Après 24 heures d'agitation à température ambiante, l'excès de borohydrure de sodium est éliminé par addition de 6 ml d'une solution d'acide chlorhydrique à 37 %. Les différents solvants sont évaporés puis le solide obtenu est repris dans 20 ml d'eau. La solution est extraite par trois fois 10 ml de dichlorométhane. Après séchage sur MgSO₄ et évaporation des solvants, le composé (VI) (R' = -CH₂-C₆H₅) est obtenu sous forme d'un solide jaune (0,23 g ; Rdt global par rapport au composé (IIb) = 41 %). RMN ¹³C (125 MHz, CDCl₃, δ en ppm) : 13,3 ; 49,0 ; 49,5 ; 50,8 ; 52,8 ; 53,5 ; 54,3 ; 55,1 ; 55,7 ; 57,0 ; 60,0 ; 60,4 ; 128,3 ; 128,4 ; 129,6 ; 129,9 ; 130,2 ; 137,9 ; 138,2.

Le composé (VII) peut être obtenu par hydrogénolyse du composé de formule (VI) dans lequel R' est un radical benzyle.

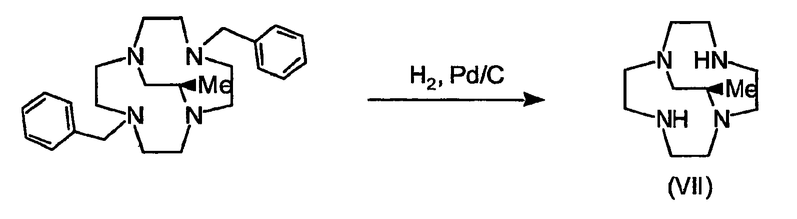

La méthode de synthèse du décahydro-2a,4a,6a,8a-tétraaza-cyclopenta[*fg*]acénaphthylène (IIa) à partir de la triéthylènetétraamine [étape (A)], est une alternative avantageuse aux procédés déjà connus de préparation de ce précurseur du cyclène.

En effet, il s'agit d'un procédé en un pot (dit "one-pot" en langue anglaise) ne nécessitant pas l'isolement du dérivé bisaminal non cyclisé intermédiaire, contrairement à tous les procédés déjà décrits. L'utilisation de dérivés dihalogénés toxiques (bibromo- ou dichloroéthane) est évitée. Un procédé récent permet également d'éviter l'utilisation de tels dérivés, mais nécessite trois étapes dont une réaction délicate de réduction d'un intermédiaire diamide. Enfin, les conditions de réactions, température ambiante, milieu concentré eau/méthanol, temps de réactions très courts, sont adaptées à un procédé de synthèse industrielle du décahydro-2a,4a,6a,8a-tétraazacyclopenta[*fg*]acénaphthylène (IIa).

Le procédé de synthèse du cyclène via l'intermédiaire (IIb) décrit dans cette invention [série d'étapes(II)], présente les mêmes avantages que ceux évoqués précédemment pour la synthèse du composé (IIa). S'y ajoute une plus grande facilité de réalisation de l'étape (D) que de l'étape (B). En effet, le traitement de (IIb) par une solution HCl 37% suffit à obtenir le cyclène avec un rendement de 50%, permettant ainsi d'éviter l'emploi de réactifs tels que l'hydroxylamine en excès ou encore des oxydants (Br₂, KMnO₄) et/ou de conditions drastiques (autoclave à 180°C, reflux d'une solution d'H₂SO₄ concentrée).

Le pont aminal a déjà été utilisé comme groupe protecteur pour fonctionnaliser sélectivement une ou deux fonctions amines en position trans d'une tétraamine cyclique²³⁻³⁰ ou pour accéder à des dérivés macrobicycliques du cyclène ou du cyclame.³¹⁻³⁹ Cependant, les dérivés de type bisaminal sont obtenus par action du formaldéhyde ou du glyoxal sur le macrocycle déjà formé. Dans cette invention, le 8b-méthyldécahydro-2a,4a,6a,8a-tétraazacyclopenta[*fg*] acénaphthylène (IIb) peut être sélectivement mono- ou diquaternisé pour conduire après hydrolyse basique à des cyclènes N-mono- ou 1,7-difonctionnalisés. Les conditions de déprotection des composés de formules (IVa), (IVa') et (IVb) sont plus douces que celles nécessaires pour hydrolyser les analogues non méthylés obtenus par utilisation du glyoxal. Les cyclènes tri-N-fonctionnalisés peuvent être aisément obtenus par fonctionnalisation du cyclène mono-N-benzylé suivie d'une réaction d'hydrogénolyse. Les agents bifonctionnels chélatants, macrocycles à très haute valeur ajoutée dont certains représentants sont utilisés comme agents de contraste en IRM, peuvent être préparés indifféremment à partir de cyclènes mono ou tri N-fonctionnalisés.

Ce procédé représente donc une voie d'accès directe à des précurseurs d'agents chélatants bifonctionnels directement au départ de l'amine linéaire, le même groupe bisaminal jouant à la fois le rôle de "template" favorisant la réaction de cyclisation et celui de groupe protecteur permettant une fonctionnalisation sélective. Cette nouvelle méthode permet donc de préparer, en un nombre limité d'étapes, des macrocycles à très haute valeur ajoutée utilisés dans le domaine médical, en particulier comme agents de contraste pour l'imagerie médicale. Enfin, il est à noter que la réduction des intermédiaires diquaternisés conduit à de nouveaux ligands macrobicycliques, analogues méthylés de composés qui ont déjà fait l'objet de plusieurs brevets.

Les références des travaux cités dans le présent exposé, sont les suivantes :
(1) Athey, P. S.; Kiefer, G. E., WO 95/14726 ; Athey, P. S.; Kiefer, G. E. J. Org. Chem. 2002, 67, 4081-4085.
(2) Sandnes, R. W.; Vasilevskis, J.; Undheim, K.; Gacek, M., WO 96/28432,
(3) Schultze, L.; Bulls, A. R., WO 96/28433,
(4) Argese, M.; Ripa, G.; Scala, A.; Valle, V., WO 97/49691,
(5) Petrov, O.; Prelle, A.; Graske, K.; Nickisch, K.; Raduchel, B.; Platzek, J., WO 97/31905,
(6) Argese, M.; Ripa, G.; Scala, A.; Valle, V., WO 98/45296,
(7) Hervé, G.; Bernard, H.; Le Bris, N.; Yaouanc, J. J.; Handel, H. Tetrahedron Lett. 1998, 39, 6861-6864.
(8) Ripa, G.; Argese, M., WO 98/49151,
(9) Sandnes, R. W.; Gacek, M.; Undheim, K. Acta Chem. Scand. 1998, 52, 1402-1404.
(10) Argese, M.; Ripa, G., WO 99/05145,
(11) Hervé, G.; Bernard, H.; Le Bris, N.; Le Baccon, M.; Yaouanc, J. J.; Handel, H. Tetrahedron Lett. 1999, 40, 2517-2520,
(12) Argese, M.; Manfredi, G.; Rebasti, F.; Ripa, G., WO 00/53588,
(13) Ferrari, M.; Giovenzana, G. B.; Palmisano, G.; Sisti, M. Synth. Commun. 2000, 30, 15-21,
(14) Hervé, G.; Bernard, H.; Toupet, L.; Handel, H. Eur. J. Org. Chem. 2000, 33-35,
(15) Platzek, J.; Hoyer, K.; Graske, K.-D.; Radüchel, B., WO 00/32581,
(16) Vasilevskis, J.; Varadarajan, J.; Garrity, M.; Fellmann, J. D.; Messerle, L.; Amarasinghe, C., US 6,048,979,
(17) Argese, M.; Brocchetta, M.; Manfredi, G.; Rebasti, F.; Ripa, G. ; WO 2001-79207
(18) Tripier, R.; Denat, F.; Guilard, R.; Ledon, H., FR 2 810 035,
(19) Liu, S.; Edwards, D. S. Bioconjugate Chem. 2001, 7-34.
(20) Denat, F.; Brandès, S.; Guilard, R Synlett, 2000, 561-574.
(21) Jacques, V.; Desreux, J.-F. in Chemistry of Contrast Agents in Medical Magnetic Resonance Imaging 2001, 157-191.
(22) Richman, J. E.; Atkins, T. J. J. Am. Chem. Soc. 1974, 96, 2268-2270.
(23) Royal, G.; Dahaoui-Gindrey, V.; Dahaoui, S.; Tabard, A.; Guilard, R.; Pullumbi, P.; Lecomte, C. Eur. J. Org. Chem. 1998, 1971-1975.
(24) Bucher, C.; Royal, G.; Barbe, J.-M.; Guilard, R. Tetrahedron Lett. 1999, 40, 2315-2318.
(25) Bucher, C.; Duval, E.; Barbe, J. M.; Verpeaux, J. N.; Amatore, C.; Guilard, R. C. R. Acad. Sci. Ser. II, 2000, 3, 211-222.
(26) Kotek, J.; Hermann, P.; Vojtisek, P.; Rohovec, J.; Lukes, I. Collect. Czech. Chem. Comm. 2000, 65, 243-266.
(27) Rohovec, J.; Gyepes, R.; Cisarova, I.; Rudovsky, J.; Lukes, I. Tetrahedron Lett. 2000, 41, 1249-1253.
(28) Kurosaki, H.; Bucher, C.; Espinosa, E.; Barbe, J.-M.; Guilard, R. Inorg. Chim. Acta 2001, 322, 145-149.
(29) Le Baccon, M.; Chuburu, F.; Toupet, L.; Handel, H.; Soibinet, M.; Dechamps-Olivier, I.; Barbier, J.-P.; Aplincourt, M. New J. Chem. 2001, 25, 1168-1174.
(30) Boschetti, F.; Denat, F.; Espinosa, E.; Guilard, R. Chem. Commun. 2002, 312-313.
(31) Weisman, G. R.; Rogers, M. E.; Wong, E. W.; Jasinski, J. P.; Paight, E. S. J. Am. Chem. Soc. 1990, 112, 8604-8605.
(32) Weisman, G. R.; Wong, E. H.; Hill, D. C.; Rogers, M. E.; Reed, D. P.; Calabrese, J. C. Chem. Commun. 1996, 947-948.
(33) Hiler, G. D., II; Perkins, C. M., WO 98/39335
(34) Hiler, G. D., II; Perkins, C. M., WO 00/32601,
(35) Wong, E. H.; Weisman, G. R.; Hill, D. C.; Reed, D. P.; Rogers, M. E.; Condon, J. S.; Fagan, M. A.; Calabrese, J. C.; Lam, K. C.; Guzei, I. A.; Rheingold, A. L. J. Am. Chem. Soc. 2000, 122, 10561-10572.
(36) Nestler, B.; Seebach, M., EP 0 940 402,
(37) Perkins, C. M., WO 2002-26748,
(38) Perkins, C. M.; Kitko, D. J., WO 2002-26267,
(39) Hubin, T. J.; Meade, T. J., WO 2002-06287,
(40) WO 03/029228
(41) WO 37/49691
(42) Hubin et al., "Inorganic chemistry," 41(26), 2002, pages 7006 - 7014.

## Revendications

1. Procédé de préparation du cyclène de formule (I) : 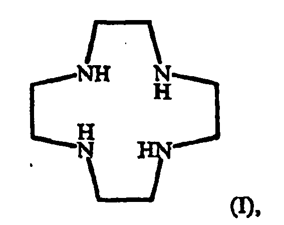 à partir de triéthylènetétraamine de formule (VIII) : 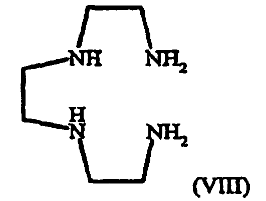 ou d'éthylènediamine de formule (VIII') : 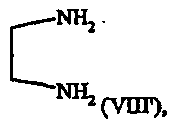 **caractérisé en ce qu'**il est constitué de l'une ou l'autre des séries d'étapes successives suivantes :
La série d'étapes (I) constituée d'une étape A de préparation en un seul pot du composé de formule (IIa) : 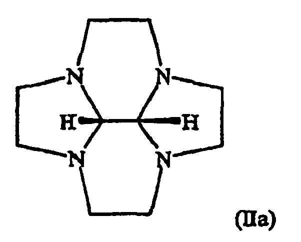 à partir du composé de formule (VIII) telle que définie précédemment, suivie d'une étape (B) de transformation du composé de formule (IIa), en cyclène de formule (I) ;
La série d'étapes (II) constituée d'une étape (C) de préparation du composé de formule (IIb) : 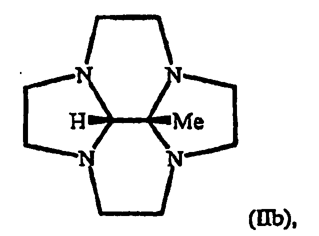 à partir du composé de formule (VIII) telle que définie précédemment, suivie d'une étape (D) de transformation du composé de formule (IIb), en cyclène de formule (I) ; ou
La série d'étapes (III) constituée d'une étape (E) de préparation en un seul pot du composé de formule (IIa) telle que définie précédemment, à partir du composé de formule (VIII') telle que définie précédemment, suivie d'une étape (B) de transformation du composé de formule (IIa), en cyclène de formule (I),
les étapes A, C et E étant **caractérisées en ce qu'**elles sont effectuées en l'absence de dérivés dihalogénés.

2. variante du procédé tel que défini à la revendication 1, dans laquelle, la série d'étapes (II) est réalisée en un seul pot.

3. Composé de formule (IIb) :

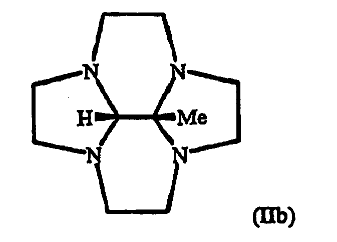

4. Procédé selon la revendication 1, **caractérisé en ce que** l'étape A de préparation du composé de formule (IIa) comprend :
- la réaction du composé de formule (VIII) avec 2 équivalents de glyoxal et 2 équivalents de benzotriazole dans de l'eau et du méthanol, suivie d'une agitation pendant 4 heures à température ambiante ; et
- l'ajout de NaBH₄ suivi d'une agitation pendant 2 heures à température ambiante.

5. Procédé selon la revendication 1 ou 4, **caractérisé en ce que** l'étape B de transformation du composé de formule (IIa) en cyclène de formule (I) correspond à l'une des étapes suivantes :
- la réaction du composé de formule (IIa) avec 10 équivalents de NH₂OH dans de l'éthanol pendant 16 heures au reflux ; ou
- la réaction du composé de formule (IIa) avec Br₂ ou KMnO₄ ou NaOCl puis l'ajout de H₂O/NaOH à une température de 150°C à 180°C en autoclave ; ou
- la réaction du composé de formule (IIa) avec Br₂ ou KMnO₄ ou NaOCl puis l'ajout de H₂SO₄ 50% pendant 24 heures à 112°C ; ou
- la réaction du composé de formule (IIa) avec 3 équivalents de Br₂ puis l'ajout de H₂O/NaOH pendant 36 heures au reflux ; ou
- la réaction du composé de formule (IIa) avec de la diéthylènetriamine et un mélange eau / HCl pendant 24 heures au reflux, suivie par des traitements successifs par HCl.

6. Procédé selon l'une quelconque des revendications 1 ou 2, **caractérisé en ce que** l'étape C de préparation du composé de formule (IIb) correspond à l'une des étapes suivantes :
- la réaction du composé de formule (VIII) avec du pyruvaldéhyde dans de l'eau pendant 2 heures à 2°C, suivie par la réaction avec du glyoxal et 2 équivalents de benzotriazole dans un mélange eau / MeOH pendant 2 heures à température ambiante, et l'ajout de NaBH₄ pendant 2 heures à température ambiante ; ou
- la réaction du composé de formule (VIII) avec du pyruvaldéhyde dans de l'eau pendant 2 heures à 2°C, suivie par la réaction avec du glyoxal et 2 équivalents de benzotriazole dans un mélange eau / EtOH pendant 2 heures à température ambiante, et l'ajout de NaBH₄ pendant 2 heures à température ambiante ; ou
- la réaction du composé de formule (VIII) avec du pyruvaldéhyde dans de l'éthanol pendant 2 heures à 0°C, suivie par la réaction avec du glyoxal et 2 équivalents de benzotriazole dans un mélange eau / EtOH, pendant 2 heures à température ambiante, et l'ajout de NaBH₄ pendant 2 heures à température ambiante.

7. Procédé selon l'une quelconque des revendications 1 ou 2 et 4 à 6, **caractérisé en ce que** l'étape D de transformation du composé de formule (IIb) en cyclène de formule (I) est une étape de réaction du composé de formule (IIb) avec du HCl 37% pendant 12 heures au reflux.

8. Procédé selon la revendication 1, **caractérisé en ce que** l'étape E de préparation du composé de formule (IIa) est une étape de réaction du composé de formule (VIII') avec 0,5 équivalent de glyoxal dans de l'eau à 2°C pendant 1 heure puis avec 1 équivalent de glyoxal et 4 équivalents de benzotriazole dans un mélange eau / méthanol pendant 2 heures à température ambiante, suivie d'une étape de réaction avec NaBH₄ pendant 2 heures à température ambiante.

## Claims

1. Process for the preparation of the cyclene of formula (I): 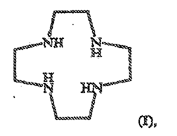 starting from triethylenetetramine of formula (VIII): 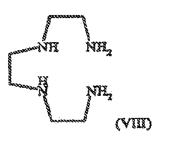 or ethylenediamine of formula (VIII'): 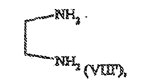 **characterised in that** it is composed of one or other of the following series of successive steps:
the series of steps (I) composed of a step A of one-pot preparation of the compound of formula (IIa): 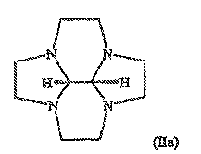 starting from the compound of formula (VIII) as defined hereinbefore, hollowed by a step (B) of conversion of the compound of formula (IIa) to the cyclene of formula (I):
the series of steps (II) composed of a step (C) of preparation of the compound of formula (IIb): 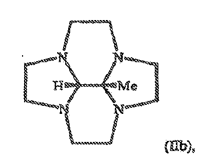 starting from the compound of formula (VIII) as defined hereinbefore, followed by a step (D) of conversion of the compound of formula (IIb) to the cyclene of formula (I): or
the series of steps (III) composed of a step (E) of one-pot preparation of the compound of formula (IIa) as defined hereinbefore, starting from the compound of formula (VIII') as defined hereinbefore, followed by a step (B) of conversion of the compound of formula (IIa) to the cyclene of formula (I),
steps A, C and E being **characterised in that** they are carried out in the absence of dihalogen compounds.

2. Variant of the process as defined in claim 1, wherein the series of steps (II) is carried out in one pot.

3. Compound of formula (IIb):

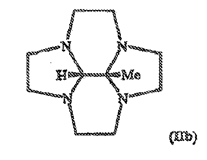

4. Process according to claim 1, **characterised in that** step A of preparation of the compound of formula (IIa) comprises:
- reaction of the compound of formula (VIII) with 2 equivalents of glyoxal and 2 equivalents of benzotriazole in water and methanol, followed by stirring for 4 hours at ambient temperature; and
- addition of NaBH₄, followed by stirring for 2 hours at ambient temperature.

5. Process according to claim 1 or 4, **characterised in that** step B of conversion of the compound of formula (IIa) to the cyclene of formula (I) corresponds to one of the following steps:
- reaction of the compound of formula (IIa) with 10 equivalents of NH₂OH in ethanol for 16 hours at reflux; or
- reaction of the compound of formula (IIa) with Br₂ or KMnO₄ or NaOCl then addition of H₂O/NaOH at a temperature of from 150°C to 180°C in an autoclave; or
- reaction of the compound of formula (IIa) with Br₂ or KMnO₄ or NaOCl then addition of H₂SO₄ 50% for 24 hours at 112°C; or
- reaction of the compound of formula (IIa) with 3 equivalents of Br₂ then addition of H₂O/NaOH for 36 hours at reflux; or
- reaction of the compound of formula (IIa) with diethylenetriamine and a water/HCl mixture for 24 hours at reflux, followed by successive treatments with HCl.

6. Process according to any one of claims 1 of 2, **characterised in that** step C of preparation of the compound of formula (IIb) corresponds to one of the following steps:
- reaction of the compound of formula (VIII) with pyruvaldehyde in water for 2 hours at 2°C, followed by reaction with glyoxal and 2 equivalents of benzotriazole in a water/MeOH mixture for 2 hours at ambient temperature, and addition of NaBH₄ for 2 hours at ambient temperature; or
- reaction of the compound of formula (VIII) with pyruvaldehyde in water for 2 hours at 2°C, followed by reaction with glyoxal and 2 equivalents of benzotriazole in a water/EtOH mixture for 2 hours at ambient temperature, and addition of NaBH₄ for 2 hours at ambient temperature; or
- reaction of the compound of formula (VIII) with pyruvaldehyde in ethanol for 2 hours at 0°C, followed by reaction with glyoxal and 2 equivalents of benzotriazole in a water/EtOH mixture for 2 hours at ambient temperature, and addition of NaBH₄ for 2 hours at ambient temperature.

7. Process according to any one of claims 1 or 2 and 4 to 6, **characterised in that** step D of conversion of the compound of formula (IIb) to the cyclene of formula (I) is a step of reaction of the compound of formula (IIb) with HCl 37 % for 12 hours at reflux.

8. Process according to claim 1, **characterised in that** step E of preparation of the compound of formula (IIa) is a step of reaction of the compound of formula (VIII') with 0.5 equivalent of glyoxal in water at 2°C for 1 hour and then with 1 equivalent of glyoxal and 4 equivalents of benzotriazole in a water/methanol mixture for 2 hours at ambient temperature, followed by a step of reaction with NaBH₄ for 2 hours at ambient temperature.

## Patentansprüche

1. Verfahren zur Herstellung des Cyclen der Formel (I): 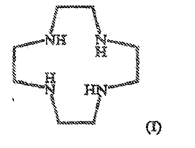 aus Triethylentetramin der Formel (VIII): 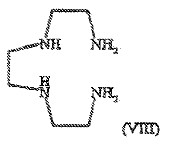 oder aus Ethylendiamin der Formel (VIII'): 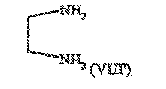 **dadurch gekennzeichnet, dass** es aus einer der folgenden Folgen von sukzessiven Stufen besteht:
Stufenfolge (I), bestehend aus einer Stufe A der Herstellung der Verbindung der Formel (IIa) in einem einzigen Topf: 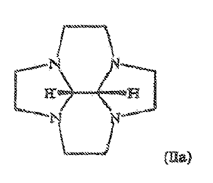 ausgehend von der Verbindung der Formel (VIII), wie sie vorstehend definiert ist, gefolgt von einer Stufe (B) der Überführung der Verbindung der Formel (IIa) in Cyclen der Formel (I);
Stufenfolge II, bestehend aus einer Stufe (C) der Herstellung der Verbindung der Formel (IIb): 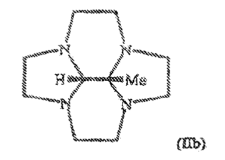 ausgehend von der Verbindung der formel (VIII), wie sie vorstehend definiert wurde, gefolgt von einer Stufe (D) der Überführung der Verbindung der Formel (IIb) in Cyclen der Formel (I); oder
Stufenfolge III, bestehend aus einer Stufe (E) der Herstellung der Verbindung der Formel (IIa), wie sie vorstehend definiert ist, in einem einzigen Topf, ausgehend von der Verbindung der Formel (VIII'), wie sie vorstehend definiert wurde, gefolgt von einer Stufe (B) der Überführung der Verbindung der Formel (IIa) in Cyclen der Formel (I),
wobei die Stufen A, C und E **dadurch gekennzeichnet sind, dass** sie in Abwesenheit von Dihalogen-Derivaten durchgerührt werden.

2. Variante des Verfahrens, wie es in Anspruch 1 definiert ist, in der die Stufenfolge (II) in einem einzigen Topf durchgeführt wird.

3. Verbindung der Formel (IIb):

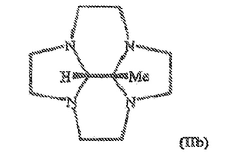

4. Verfahren gemäß Anspruch 1, **dadurch gekennzeichnet, dass** die Stufe A der Herstellung der Verbindung der Formel (IIa) umfasst:
- Reaktion der Verbindung der Formel (VIII) mit 2 Äquivalenten Glyoxal und 2 Äquivalenten Benzotriazol in Wasser und Methanol, gefolgt von einem Rühren während 4 Stunden bei Umgebungstemperatur, und
- Zusetzen von NaBH₄, gefolgt von einem Rühren während 2 Stunden bei Umgebungstemperatur.

5. Verfahren gemäß Anspruch 1 oder 4, **dadurch gekennzeichnet, dass** die Stufe B der Überführung der Verbindung der Formel (IIa) in Cyclen der Formel (I) einer der folgenden Stufen entspricht:
- Reaktion der Verbindung der Formel (IIa) mit 10 Äquivalenten NH₂OH in Ethanol während 16 Stunden unter Rückfluss oder
- Reaktion der Verbindung der Formel (IIa) mit Br₂ oder KMnO₄ oder NaOCl, danach Zusatz von H₂O/NaOH bei einer Temperatur von 150 °C bis 180 °C im Autoklaven oder
- Reaktion der Verbindung der Formel (IIa) mit Br₂ oder KMnO₄ oder NaOCl, danach Zusatz von 50 % H₂SO₄ während 24 Stunden bei 112 °C oder
- Reaktion der Verbindung der Formel (IIa) mit 3 Äquivalenten Br₂, danach Zusatz von H₂O/NaOH während 36 Stunden unter Rückfluss oder
- Reaktion der Verbindung der Formel (IIa) mit Diethylentriamin und einem Gemisch aus Wasser / HCl während 24 Stunden unter Rückfluss, gefolgt von sukzessiven Behandlungen durch HCl.

6. Verfahren gemäß einem der Ansprüche 1 oder 2, **dadurch gekennzeichnet, dass** die Stufe C der Herstellung der Verbindung der Formel (IIb) einer der folgenden Stufen entspricht:
- Reaktion der Verbindung der Formel (VIII) mit Pyruvaldehyd in Wasser während 2 Stunden bei 2 °C, gefolgt von der Reaktion mit Glyoxal und 2 Äquivalenten Benzotriazol in einem Gemisch aus Wasser / MeOH während 2 Stunden bei Umgebungstemperatur und Zusatz von NaBH₄ während 2 Stunden bei Umgebungstemperatur oder
- Reaktion der Verbindung der Formel (VIII) mit Pyruvaldehyd in Wasser während 2 Stunden bei 2 °C, gefolgt von der Reaktion mit Glyoxal und 2 Äquivalenten Benztriazol in einem Gemisch aus Wasser / EtOH während 2 Stunden bei Umgebungstemperatur und Zusatz von NaBH₄ während 2 Stunden bei Umgebungstemperatur oder
- Reaktion der Verbindung der Formel (VIII) mit Pyruvaldehyd in Ethanol während 2 Stunden bei 0 °C, gefolgt von der Reaktion mit Glyoxal und 2 Äquivalenten Benzotriazol in einem Gemisch aus Wasser / EtOH während 2 Stunden bei Umgebungstemperatur und Zusatz von NaBH₄ während 2 Stunden bei Umgebungstemperatur.

7. Verfahren gemäß einem der Ansprüche 1 oder 2 und 4 bis 6, **dadurch gekennzeichnet, dass** die Stufe D der Überführung der Verbindung der Formel (IIb) in Cyclen der Formel (I) eine Stufe der Reaktion der Verbindung der Formel (IIb) mit 37 % HCl während 12 Stunden unter Rückfluss ist.

8. Verfahren gemäß Anspruch 1, **dadurch gekennzeichnet, dass** die Stufe E der Herstellung der Verbindung der Formel (IIa) eine Stufe der Reaktion der Verbindung der Formel (VIII') mit 0,5 Äquivalenten Glyoxal in Wasser bei 2 °C während 1 Stunde, danach mit 1 Äquivalent Glyoxal und 4 Äquivalenten Benzotriazol in einem Gemisch aus Wasser / Methanol während 2 Stunden bei Umgebungstemperatur, gefolgt von einer Stufe der Reaktion mit NaBH₄ während 2 Stunden bei Umgebungstemperatur ist.
